# NEUE EUROPÄISCHE PATENTSCHRIFT

(11) **EP 0 302 357 B2**
(45) Veröffentlichungstag und Bekanntmachung des Hinweises auf die Entscheidung über den Einspruch: **18.10.1995**
(45) Hinweis auf die Patenterteilung: 05.05.1993
(21) Anmeldenummer: 88111998.6
(22) Anmeldetag: 26.07.1988
(51) Int. Cl.: A61J 1/00, A61J 1/10, B32B 27/34, A61L 2/06, A61L 2/26, B65D 30/08

(54) **Verpackungseinheiten für medizinische Zwecke**
Packaging units for medical use
Unités d'emballage à usage médical

(30) Priorität: 06.08.1987 DE 3726064
(43) Veröffentlichungstag der Anmeldung: 08.02.1989
(73) Patentinhaber: Fresenius AG, 61350 Bad Homburg (DE)
(72) Erfinder: Sommermeyer, Klaus, Dr., D-6365 Rosbach v.d.H. (DE); Koenig, Jürgen, D-6200 Wiesbaden (DE); Cech, Franz, Dr., D-6365 Rosbach v.d.H. (DE); Herbert, Reinhold, D-6392 Neu-Anspach (DE)
(74) Vertreter: Fuchs Mehler Weiss

(56) Entgegenhaltungen:
- WO-A-86/02042
- CA-A- 922 614
- DE-A- 2 949 789
- DE-A- 3 200 264
- DE-A- 3 218 415
- DE-A- 3 300 944
- DE-U- 8 304 946
- US-A- 4 331 786

## Beschreibung

Gegenstand der vorliegenden Erfindung sind Verpackungseinheiten für medizinische Zwecke, insbesondere zur Aufnahme und/oder Lagerung von sterilisierbaren Parenteralien oder Dialyseflüssigkeiten, nach Teil 1 des Anspruchs 1.

Die Verwendung von Verpackungseinheiten, wie zum Beispiel Beutel, insbesondere für medizinische Zwecke zur Lagerung von Parenteralien oder Dialyseflüssigkeiten, anstelle von Glasflaschen, zur Aufnahme und zur sterilen Lagerung von parenteral zu verabreichenden Infusionslösungen oder von Dialyseflüssigkeiten, ist seit langem bekannt. Zu diesem Zweck müssen diese Beutel völlig steril sein, was üblicherweise durch ein Erhitzen auf wenigstens 100°C, insbesondere etwa 120°C erzielt wird.

Hieraus ergibt sich bereits die Anforderung an das thermoplastische polymere Material, wenigstens bis zur Erhitzungstemperatur stabil zu sein. Darüber hinaus muß ein derartiger Aufbewahrungsbeutel leicht, billig und in einem automatischen Herstellungsprozeß herzustellen sein und wegen seines geringen Werts nach Gebrauch wegwerfbar sein. Weiterhin soll er biegbar, zusammenfaltbar und im verarbeiteten Zustand derart transparent sein, daß Veränderungen der im Beutel enthaltenen Flüssigkeit sofort bemerkt werden können.

Als Material, das diese Voraussetzungen erfüllte, wurde bevorzugt Weich-PVC, das zur Verbesserung seiner elastischen Eigenschaften Weichmacher enthält, eingesetzt. Diese Weichmacher, beispielsweise Diisooctylphthalat, geben jedoch Anlaß zu bedenklichen Erscheinungen. Diese Weichmacher werden nicht vollständig in den Hohlräumen zwischen den polymeren Ketten eingeschlossen und können daher durch das in den Beutel eingefüllte Wasser bzw. die wäßrige Lösung aus dem Polymerisat herausgelöst werden, so daß es zu einer Verunreinigung der im Beutel enthaltenen Flüssigkeit kommt. Schätzungen haben ergeben, daß ein über längere Zeit unter Verwendung derartiger PVC-Beutel behandelter Patient einige g Weichmacher in sich aufgenommen hat, was an sich physiologisch äußerst bedenklich ist und zu dauerhaften Schäden bei dem Patienten führen kann. Darüber hinaus wird ein derartiger aus Weich-PVC bestehender Beutel leicht von Mikroorganismen befallen, die insbesondere die Weichmacher herauslösen und damit regelmäßig den Beutel zerstören. Um dies zu verhindern, mußte ein derartiger PVC-Beutel nach dem Füllen mit einer speziellen Umverpakkung vor schädigenden Organismen geschützt werden.

Diese Tatsachen führten dazu, daß derartige PVC-Beutel die üblicherweise eingesetzen Glasflaschen als Aufbewahrungsbehälter für medizinische Lösungen nicht in wesentlichem Umfang verdrängen konnten und teilweise sogar in einigen Industrieländern auf dem medizinischen Gebiet überhaupt nicht zugelassen sind.

Es wurden daher Versuche unternommen, das Weich-PVC durch andere Stoffe zu ersetzen. Diese Versuche scheiterten jedoch daran, daß diese Stoffe entweder zu teuer waren oder aber mechanische und physiologische Nachteile aufwiesen. Beispielsweise besaßen sie eine zu hohe Wasserdampfdurchlässigkeit, was zur unerwünschten Erhöhung der Konzentration der in der Lösung enthaltenen Stoffe führte. Darüber hinaus setzten sie auslaugbare Bestandteile frei oder waren bei zu hoher mechanischer Beanspruchung leicht zu beschädigen.

Gemäß der CH-A-444 382 wird ein derartiger Kunststoffbeutel beschrieben, der für parenteral zu verwendende therapeutische Flüssigkeiten eingesetzt werden kann. Bei diesem Kunststoffbeutel besteht die Wandung aus einem Kunststofflaminat, das auf der Außenseite, also der flüssigkeitsabgewandten Seite, eine PVC-Schicht und auf der Innenseite eine Polyhalogenkohlenwasserstoff-Kunstharzschicht aufweist. Letztere Schicht besitzt keine pharmakologisch unzulässigen Bestandteile, die durch Auflösen in die in dem Beutel enthaltene Lösung übergehen könnten. Die eingesetzten Polyhalogenkohlenwasserstoffe haben jedoch den Nachteil, daß sie sehr kostenaufwendig zu produzieren und zu verarbeiten sind und an den Schweißnähten nicht ausreichend verschmelzen, so daß noch ein direkter Kontakt mit dem PVC besteht. Dieser Kontakt besteht im übrigen auch an der Auslauföffnung, die üblicherweise vollständig aus PVC ausgeführt ist und mit den weiteren PVC-Verbindungsschläuchen verbunden werden kann. Dieser Kunststoffbeutel stellt als Wegwerfbeutel darüber hinaus eine Belastung für die Umwelt dar, da die Verbrennung dieser Beutel zu hochaggressiven Kohlenwasserstoffen führt.

Auch Polyolefine, z. B. Polyethylen, wurden bereits als Materialien für die Aufbewahrungsbeutel vorgeschlagen. Polyolefine sind weichmacherfrei und werden so nicht von Mikroorganismen angegriffen. Darüber hinaus besitzen sie eine gute Wasserdampfsperre und sind sie sterilisierbar. Beutel aus Polyolefinen werden z. B. in der DE-A-32 00 264 und der DE-A-33 05 365 beschrieben.

Diese Beutel aus Polyolefinen haben jedoch leider den Nachteil, daß sie eine relativ hohe Sauerstoffdurchlässigkeit aufweisen, was bei der Lagerung von Lösungen, die über längere Zeit aufbewahrt werden müssen, insofern problematisch ist, als es infolge der Sauerstoffdurchlässigkeit zur Oxidation der gelösten Bestandteile kommen kann. Letzteres ist insbesondere bei Aminosäurelösungen äußerst kritisch und muß daher vermieden werden. Zur Behebung dieses Problems wurde gemäß der DE-PS 32 00 264 und der DE-PS 33 05 365 vorgeschlagen, die Beutelfolie aus dem Polyolefin auf ihrer Außenseite mit einer oder mehreren, diese Sauerstoffdurchlässigkeit senkenden Schicht(en), beispielsweise einer Metallfolie oder einem weiteren Polymerisat zu beschichten. Eine solche Beschichtung wird auch aus Sicherheitsgründen eingesetzt, da selbst bei äußerst sorgfältiger Herstellung die Beutelfolie nadelförmige Löcher (pin holes) aufweist, die nicht zu erkennen sind und die Sterilität der eingefüllten Lösung beeinträchtigen können. Darüber hinaus kann eine solche Überschichtung auch die mechanische Belastbarkeit eines derartigen Beutels erheblich verbessern, so daß ein solcher Beutel auch bei einem Wurf aus einer Höhe von mehreren Metern nicht zerplatzt. Als Folie oder Schicht wird dabei eine solche eingesetzt, die einen höheren Schmelzpunkt als das der Lösung zugewandte Polymerisat aufweist, die also bei der Schmelztemperatur der Innenfolie selbst nicht schmilzt und infolgedessen auch an einem Siegelwerkzeug nicht anhaftet. Eine solche Außenfolie kann daher als Trennmittel bei der Versiegelung der Innenfolie dienen. Als bevorzugte Polymere zur Beschichtung der Polyolefinfolie werden solche mit einer niedrigen Wasserdampfdurchlässigkeit und einer niedrigen Sauerstoffdurchlässigkeit, wie Polyamide, genannt, wobei als Polyamid Polycaprolactam (PA 6), das keine stabilisierenden Zusätze enthält und somit in seiner Zusammensetzung den Anforderungen zum Einsatz auf dem Lebensmittelsektor entspricht, bevorzugt wird.

Wie jedoch jüngste Untersuchungen an Beuteln aus solchen Polyolefin/Polyamidlaminaten gezeigt haben, weisen die Laminate aus Polyolefin und Polyamid, wie Laminate aus Polyethylen und Polycaprolactam, Nachteile derart auf, daß in die aufzubewahrenden Flüssigkeiten bei der Sterilisierung unerwünschte toxische Fremdbestandteile freigesetzt werden, die die Lösung für ihre Verwendung, z. B. für die Injektion, unbrauchbar machen können. Diese Fremdbestandteile sind - wie festgestellt werden konnte - durch Migration aus der Außenfolie des Laminates durch die der Flüssigkeit zugewandte Innenfolie des Laminates in die aufzubewahrende Flüssigkeit gelangt.

Beutel aus derartigen Laminaten sind daher vom medizinischen Standpunkt höchst bedenklich.

Demzufolge liegt der vorliegenden Erfindung die Aufgabe zugrunde, eine Verpackungseinheit der eingangs erwähnten Art bereitzustellen, die die Nachteile der bekannten Verpackungseinheiten bzw. Beutel nicht aufweisen, vom medizinischen Standpunkt unbedenklich sind und bei denen keine Migration von Fremdbestandteilen in die aufzubewahrende Lösung auftritt, die mechanisch stabil, klarsichtig und hitzesterilisierbar sind und niedrige Wasserdampfdurchlässigkeit und niedrige Sauerstoffdurchlässigkeit aufweisen und darüber hinaus von Mikroorganismen nicht befallen werden können.

Erfindungsgemäß wird diese Aufgabe durch Teil 2 des Anspruchs 1 gelöst.

Unter dem Ausdruck "polymeres, Polyamid aufweisendes Material" wird hierin Polyamid oder Polyamid aufweisendes Laminat aus polymerem Material verstanden. So kann erfindungsgemäß der Behälter der erfindungsgemäßen Verpackungseinheit aus einem Polyamid 66 aufweisendem Laminat aus polymerem Material bestehen.

Unter Polyamid 66 (PA 66) wird das durch Polykondensation von Hexamethylendiamin und Adipinsäure erhaltene Polykondensat verstanden.

Erfindungsgemäß wurde überraschend gefunden, daß bei Verwendung von Polyamid 66 - im Gegensatz zu den anderen Polyamiden, wie z. B. Polyamid 6, Polyamid 11, Polyamid 12 oder Polyamid 13 - als polymeres Material in Behältern für die Verpackungseinheit nach dem Sterilisieren keine Fremdbestandteile bzw. keine wägbaren Mengen an Fremdbestandteilen in der aufzubewahrenden Flüssigkeit zu finden sind; wenn es als polymeres Material für die Behälter der Verpackungseinheit Polyamid 66 im Laminat mit Polyolefinfolien, wobei auf der der aufzubewahrenden Flüssigkeit zugewandten Seite die Polyolefinfolie und auf der Außenseite die Folie aus Polyamid 66 angeordnet sind und wobei die beiden Folien in üblicher Weise mittels eines Klebers verbunden sind, verwendet wird.

Während z. B. bei Verwendung von Beuteln aus einem Laminat aus Polyethylen und Polyamid 6, wobei die Polyethylenfolie als Innenfolie und die Polyamid 6-Folie als Außenfolie verwendet wurde, nach dem Sterilisieren in der aufzubewahrenden Flüssigkeit Fremdbestandteile in einer Menge von 5 bis 15 ppm, bezogen auf das Produkt, gefunden wurden, wurden unter den gleichen Bedingungen für Beutel, die aus einem Laminat aus Polyethylen und Polyamid 66 hergestellt wurden, wobei sich die Polyethylenfolie auf der der Flüssigkeit zugewandten Seite und die Polyamid 66-Folie auf der Außenseite befand, in der aufzubewahrenden Flüssigkeit keine bzw. keine wägbaren Mengen (Wägegenauigkeit < 1 mg) an Fremdbestandteilen gefunden.

Gemäß einer bevorzugten Ausführungsform wird als Material für den Behälter der erfindungsgemäßen Verpackungseinheit das Polyamid 66 im Laminat mit einem Polyolefin verwendet, wobei auf der der aufzubewahrenden Flüssigkeit zugewandten Seite die Polyolefinfolie und auf der Außenseite die Polyamid 66-Folie angeordnet ist und die beiden Folien in üblicher Weise mittels eines Klebers verbunden sind.

Als Polyolefine, die als Polymere für die Innenfolie zur Herstellung der erfindungsgemäßen Verpackungseinheit eingesetzt werden können, sind erfindungsgemäß Polymerisate aus Olefinen, wie Ethylen, Propylen, Butylen und dergl., die gegebenenfalls substituiert sein können, geeignet. Als Substituenten können beispielsweise die Methyl- oder Ethylgruppe, die Vinylgruppe und Halogenatome, insbesondere Fluor- oder Chloratome vorliegen. Bevorzugt als Ausgangsolefin wird vorzugsweise Ethylen und Propylen verwendet, wobei insbesondere Ethylen, das zu Polyethylen polymerisiert ist, verwendet wird.

Spezielle Beispiele für Polyolefine sind: Polyethylen, Polypropylen, Poly-n-butylen, Polyisobutylen, Poly-4-methylpenten-1, chlorsulfoniertes Polyethylen, Polystyrol, halogenierte Polyethylene, wie Polyvinylfluorid, Polyvinylidenfluorid und Polyvinylidenchlorid, Polymethylmethacrylat u. dgl. Die zur Herstellung vorstehender Polyolefine eingesetzten Olefine können auch als Copolymerisate und Mischpolymerisate mit anderen Vinylverbindungen eingesetzt werden, beispielsweise Ethylen/Propylen-Kunststoffe, Poly-(Ethylen/Vinylacetat), Acrylnitril/Butadien/Styrol-Polymerisate, Ethylen-Propylen-Block-Copolymerisate, Styrol-Copolymerisate, Vinylidenfluorid enthaltende Copolymerisate sowie Styrol enthaltende Copolymerisate.

Polyolefine sind erfindungsgemäß Produkte, die durch die Vinylpolymerisation von gegebenenfalls substituiertem Olefin, vorzugsweise Ethylen, hergestellt werden. Diese Produkte können auch geringe Zusätze von weiteren Polymerisaten aufweisen, die im wesentlichen nicht die Struktur dieser Polyolefine stören oder verändern. So können beispielsweise geringe Zusätze von Styrol oder Polyacrylnitril substituierten Ethylenverbindungen zugesetzt werden. Die dadurch entstehenden Polyolefin-Produkte werden als zu den vorstehend genannten Polyolefinen zugehörig angesehen.

Erfindungsgemäß wird als Polyolefin vorzugsweise Polyethylen als Material für die Innenfolie eingesetzt, das gegebenenfalls geringe Zusätze an Vinylacetat in Form des Copolymers von Ethylen und Vinylacetat aufweisen kann.

In einem solchen Fall kann beispielsweise der Vinylacetatgehalt bis zu 10 Gew.-% betragen.

Insbesondere wird ein Polyethylen mit mittlerer bis hoher Dichte (MDPE und HDPE) eingesetzt, das üblicherweise durch Niederdruckpolymerisation hergestellt wird. Dabei liegt die Dichte in einem Bereich von 0,91 bis 0,94 g/cm³, insbesondere etwa bei 0,935 g/cm³.

Weiterhin weist das erfindungsgemäß bevorzugt eingesetzte Polyethylen ein hohes Molekulargewicht und eine enge Molekulargewichtsverteilung auf.

In jedem Fall ist jedoch darauf zu achten, daß derartige Polyolefine keinen Schmelzpunkt besitzen, der unterhalb der Sterilisationstemperatur von etwa 110 - 120°C liegt. Vorzugsweise soll der Schmelzbereich oberhalb 110°C liegen.

Für die Herstellung der für die Laminate geeigneten Folien können die üblichen Extrudierverfahren zur Herstellung von Folien oder Schlauchfolien, die dem Fachmann keine nennenswerten Probleme aufgibt, angewandt werden. Die polymere Innenfolie und die polymere Außenfolie werden in an sich bekannter Weise verbunden. Jede bekannte Methode, die zur Herstellung der erfindungsgemäß angewandten Laminate geeignet ist, kann angewendet werden. Vorzugsweise werden die Innen- und Außenfolien mittels eines Kaschierklebers, wie Polyvinylidenchlorid oder eines Polyurethans zusammengeklebt. Ein derartiger Polyurethan-Kleber kann vorteilhafterweise ein 2-Komponentenklebstoff sein, wobei die erste Komponente aus einem Kaschierkleber und die zweite Komponente aus einem Zusatzlack besteht.

Herstellungstechnisch kann das als Innenfolie eingesetzte Polyolefin in Form einer schlauchartigen Folie extrudiert und anschließend mit der Kaschierfolie aus Polyamid 66 unter Verwendung der vorstehend erwähnten Kaschierkleber, wobei Polyurethan bevorzugt ist, verklebt werden.

Bevorzugte Laminate weisen bei einer Temperatur von etwa 23° C und einer relativen Luftfeuchtigkeit von 85 % in der Regel eine Wasserdampfdurchlässigkeit nach DIN 53 122 von < 1 auf. Derartige Werte gelten für Standardlaminate, die vorteilhafterweise bis zu 0,2 mm stark sind, mit einer Stärke von 50 bis 150 µm, insbesondere etwa 100 µm für die Polyolefinfolie und 20 bis 100 µm, insbesondere 30 bis 80 µm für die polymere Kaschierfolie aus Polyamid 66. Beispielsweise besteht ein besonders geeignetes Laminat aus einer 130 µm starken Polyethylenfolie und einer 50 µm starken Folie aus Polyamid 66.

Bei den erfindungsgemäßen Laminaten, insbesondere bei den bevorzugten Laminaten, ist die Sauerstoffdurchlässigkeit gesenkt, wobei dieser Wert unterhalb 15 cm³/m² x Tag x bar Druckdifferenz liegt.

Die erfindungsgemäß verwendeten Folien zur Herstellung des Laminates sind sowohl vom Bundesgesundheitsamt in Berlin als auch von der FDA (Federal Drug Administration) in den USA wegen ihrer physiologischen Unbedenklichkeit zur Verwendung im Lebensmittelbereich und im medizinischen Bereich zugelassen worden.

Die für die erfindungsgemäßen Verpackungseinheiten eingesetzten Laminate aus Polyolefin und Polyamid 66 sind frei von Weichmachern und Zusätzen oder Bestandteilen, die u. U. physiologisch bedenklich sind und vor allen Dingen in die wäßrige Lösung diffundieren bzw. migrieren könnten. Die erfindungsgemäß verwendeten Laminate sind hitzesterilisierbar, klar und durchsichtig und weisen diese Eigenschaften auch nach dem Sterilisieren auf.

Ferner sind sie mechanisch stabil und weisen niedrige Wasserdampfdurchlässigkeit und eine hohe Sauerstoffsperre auf.

Die erfindungsgemäßen Verpackungseinheiten bzw. die Behälter dieser erfindungsgemäßen Verpackungseinheiten können jede geeignete Form bzw. Gestalt aufweisen. Geeigneterweise sind sie beutelartig ausgebildet.

Die erfindungsgemäßen Verpackungseinheiten bzw. Behälter oder Beutel weisen wenigstens eine Auslauftülle auf, die schlauchartig ausgebildet ist bzw. ein Rohrstück oder Einsatzstück umfaßt. Letztere können aus jeweils geeignetem Material bestehen und können in beliebiger geeigneter Weise gestaltet und im Behälter oder Beutel befestigt sein. sein. Beispielsweise können sie so wie in der DE-A-33 05 365 und der DE-A-32 00 264, auf die hierin Bezug genommen wird, beschrieben, gestaltet sein und aus den dort genannten Materialien bestehen.

Die Herstellung und Verarbeitung der Materialien für die erfindungsgemäßen Verpackungseinheiten bzw. Behälter erfolgt, wie vorstehend bereits beschrieben, nach den in der Kunststofftechnik üblichen Methoden. So können beispielsweise die Behälter aus einer Extrusion schlauchförmiger Folien, ensprechendem Zuschnitt der Folien und Laminierung und anschließender Randschweißung hergestellt werden. Die Behälter, bei denen der für die Auslauftülle vorgesehene Rand unverschweißt bleibt, werden anschließend mit einem gegebenenfalls eine Verbindungsschicht aufweisendem Rohrstück oder einem die Auslauföffnung(en) aufweisenden und gegebenenfalls von einer Verbindungsschicht umgebenen Einsatzstück verschmolzen. Bei Verwendung einer Verbindungsschicht, wie sie beispielsweise in der DE-PS 33 05 365 beschrieben ist, werden das Rohr- bzw. Einsatzstück mit einer solchen Verbindungsschicht gemäß üblichen Techniken versehen, was z. B. durch einfaches Aufziehen oder Aufstülpen des elastischen Materials der Verbindungsschicht in Schlauchform auf das Rohrstück oder Einsatzstück erfolgen kann, und in den geöffneten Behälter eingeführt. Anschließend erfolgt die Hitzesiegelung des gesamten, noch nicht verschweißten Randes mit dem Schlauchansatzstück bzw. dem mit der Verbindungsschicht versehenen Rohrstück oder Einsatzstück. Sofern mehrere Rohrstücke vorgesehen sind, wird dieser Bearbeitungsschritt gleichzeitig ausgeübt, wobei natürlich entsprechende Siegelwerkzeuge benutzt werden. Die Hitzesiegelung wird gemäß üblichen Verfahren durchgeführt.

Die Sterilisierung der so hergestellten Behälter erfolgt nach üblichen Methoden im Autoklaven, wobei natürlich, um das Platzen der Behälter zu vermeiden, im Autoklaven zum Ausgleich des im Behälterinneren herrschenden Druckes ein Überdruck angewandt werden muß. Aus Sicherheitsgründen und - im Falle der Verwendung einer Verbindungsschicht - zur Fixierung der Verbindungsschicht, wird jedoch ein höherer Überdruck angewandt, als er zum Ausgleich des im Behälterinneren herrschenden Druckes notwendig ist. Die Höhe dieses Überdruckes über den im Behälter herrschenden Druck ist nicht kritisch, z. B. sollte er mindestens 0,5 bar größer als der im Behälter herrschende Druck sein. Er kann 2 bis 3 bar, beispielsweise 2,2 bar betragen.

Die erfindungsgemäßen Laminate können ferner nach dem Verschließen der Behälter - bevor die aufzubewahrende Flüssigkeit eingefüllt wird und vor dem Sterilisieren - noch einer Kreuzvernetzung, falls dies aufgrund der eingesetzten Materialien notwendig ist, unterworfen werden.

In den Fällen, wo eine Kreuzvernetzung des verschweißten, Auslauftüllen mit Schlauchansatzstücken bzw. Rohr- oder Einsatzstücken aufweisenden Behältermaterials vorgesehen ist, wird diese Kreuzvernetzung vor dem Sterilisieren - falls eine Sterilisierung dann noch erforderlich ist - nach an sich bekannten Verfahren, wie sie beispielsweise in der DE-PS 32 00 264 und der EP-PS 0 068 271 beschrieben sind, durchgeführt.

Gemäß einer weiteren Ausführungsform kann, insbesondere in Fällen, wo ein besonders hoher Schutz gegen mechanische Beschädigung erzielt werden soll und/oder für die Langzeitlagerung der Verpackungseinheiten, der Behälter der erfindungsgemäßen Verpackungseinheit von einem weiteren Behälter, einem Außenbehälter, umgeben sein.

Geeignete Materialien für einen solchen weiteren Behälter oder Außenbehälter sind solche, die den erfindungsgemäßen Behälter, der bei Verwendung eines Außenbehälters den Innenbehälter darstellt, gegenüber mechanischer Beschädigung zu schützen vermögen, eventuelle Wasserdampfverluste vermeiden, Mikroorganismen keinen Zutritt (keine Verpilzung oder Versporung) gestatten, eine Gassperre, d. h. Sauerstoffsperre, darstellen, vorzugsweise im wesentlichen klarsichtig sind und eine Langzeitlagerung der Verpackungseinheiten erlauben.

Beispiele für solche Materialien sind Metall- oder Kunststoffolien oder -laminate, die die vorstehend genannten Bedingungen erfüllen. Bevorzugt sind Folien oder Laminate aus Kunststoff.

Beispiele für Metallfolien oder -laminate sind:
Aluminiumfolien,
Laminate aus Polyethylenfolien und Aluminiumfolien,
Laminate aus Polypropylenfolien und Aluminiumfolien.

Als Kunststoffe geeignet sind im wesentlichen die Polyolefine, wie sie vorstehend im Zusammenhang mit den erfindungsgemäßen Behältern, die bei Verwendung von Außenbehältern die Innenbehälter darstellen, genannt wurden. Diese Kunststoffe können in Form von Einzelfolien oder in Form von Laminaten aus zwei oder mehreren Folien verwendet werden. Laminate aus 2 Folien: Innenfolie, d. h. der dem Innenbehälter zugewandten Folie, und Außenfolie, d. h. der äußeren, mit der Umgebung in Berührung kommenden Folie, werden bevorzugt.

Insbesondere geeignet sind erfindungsgemäß für den Außenbehälter Laminate aus Polyolefinen, wie sie vorstehend für den Innenbehälter genannt wurden, wobei Polyethylen als Polyolefin besonders bevorzugt wird, und Polyester, Copolymere aus Ethylen und Vinylalkohol, Copolymere aus Ethylen und Vinylacetat. Beispiele für geeignete Laminate sind Laminate aus Polyethylen-Folie und Polyester-Folie, wobei als Polyester Polyethylenterephthalat und Polybutylenterephthalat besonders geeignet sind, Laminate aus Polyethylenfolie und Folie aus Copolymeren von Ethylen und Vinylalkohol, Laminate aus Polyethylen-Folie und Folie aus Copolymeren aus Ethylen und Vinylacetat.

Mit diesem Außenbehälter wird erfindungsgemäß ferner eine Doppelsauerstoffsperre bereitgestellt und werden die Behälterinhalte, d. h. die aufzubewahrende Flüssigkeit oder Lösung, vor Oxidation geschützt.

Bei der Herstellung dieser erfindungsgemäßen, Außenbehälter-aufweisenden Verpackungseinheiten wird zunächst der Innenbehälter - wie vorstehend beschrieben - hergestellt, wobei im Falle der Verwendung eines Laminates aus einer Polyamid 66-Folie und einer Polyolefinfolie auf der der aufzubewahrenden Flüssigkeit zugewandten Seite die Polyolefinfolie und auf der Außenseite die Folie aus Polyamid 66 angeordnet ist und die beiden Folien mittels eines Klebers verbunden sind. Anschließend wird der so hergestellte Innenbehälter - nach gegebenenfalls erfolgter Kreuzvernetzung - mit der aufzubewahrenden Flüssigkeit oder Lösung gefüllt, verschlossen und,wie vorstehend beschrieben, sterilisiert. Nach Erkalten und Abtrocknen dieses sterilisierten Innenbehälters wird der mit der aufzubewahrenden Flüssigkeit gefüllte, geschlossene und sterilisierte Innenbehälter in an sich bekannter Weise mit dem Außenbehälter versehen.

So wird beispielsweise der sterilisierte, abgetrocknete und erkaltete Innenbehälter in eine genau seinen Konturen entsprechende tiefgezogene Unterbahn für den Außenbehälter gelegt und mit der Oberbahn für den Außenbehälter bedeckt. Danach wird an den Außenbehälter ein Vakuum angelegt, das etwa 900 bis 980 mbar betragen sollte. Gleichzeitig oder unmittelbar anschließend daran werden die Ränder von Oberbahn und Unterbahn des Außenbehälters miteinander verschweißt. Wenn das Vakuum angelegt wird, legt sich die Folie des Außenbehälters dicht an das Laminat des Innenbehälters an.

Die Unterbahn und die Oberbahn können aus gleichem oder verschiedenem Material bestehen und bestehen vorzugsweise aus gleichem Material. Ebenso können die Oberbahn und die Unterbahn des Außenbehälters gleiche oder verschiedene Stärken aufweisen und sind vorzugsweise gleich stark. Wesentlich ist, daß die Materialien von Unterbahn und Oberbahn - bei Laminaten die Innenschichten von Unterbahn und Oberbahn - miteinander verschweißbar sind. Wird nur die Unterbahn tiefgezogen, so sollte die Unterbahn vorzugsweise etwas stärker sein als die Oberbahn. Geeigneterweise weisen die Oberbahn und die Unterbahn Stärken von 100 bis 200 µm auf.

Gemäß einer weiteren Ausführungsform der erfindungsgemässen Verpackungseinheiten kann der vorstehend genannte, aus Polyamid 66 oder einem polymeren, Polyamid 66 aufweisenden Laminat bestehende Behälter den Außenbehälter darstellen und einen wenigstens eine Auslauftülle aufweisenden Innenbehälter aus polymerem Material umgeben, wobei im Falle der Verwendung eines Polyamid 66 aufweisenden Laminates für den Außenbehälter die dem Innenbehälter zugewandte Seite des Laminates aus Polyolefinfolie und die Außenseite des Laminates aus Polyamid 66- Folie besteht und die beiden Folien in üblicher Weise mittels eines Klebers verbunden sind.

Als Material für den Innenbehälter gemäß dieser Ausführungsform ist jedes polymere Material geeignet, das sich für die Aufbewahrung und Lagerung der sterilisierbaren Parenteralien oder Dialyseflüssigkeiten eignet, eine Sterilisierung erlaubt und keine migrierbaren Stoffe aufweist. Das Behältermaterial kann in Form von Einzelfolien oder in Form von Laminaten vorliegen und ist vorzugsweise im wesentlichen klarsichtig. Beispiele für solche Materialien sind: Polyolefine, Polyester und Copolymere derselben. Zu den Polyolefinen gehören die vorstehend genannten Polyolefine, einschließlich PVC und Weich-PVC, wobei Polyethylen bevorzugt wird. Als Polyester geeignet ist beispielsweise Polyethylenterephthalat.

Als Material für die Innenbehälter gemäß dieser Ausführungsform insbesondere bevorzugt sind - in Form von Einzelfolien oder von Laminaten - Polyethylen, Copolymerisate aus Ethylen und Propylen und Polyester, wie Polyethylenterephthalat.

Als Laminate geeignet sind Verbundfolien aus den vorstehend genannten Materialien, gegebenenfalls mit eingebetteten Ethylen/Vinylalkohol (EVAL)-Sperrschichten. Beispiele für geeignete Laminate sind Laminate aus Polyolefin-Folien, besonders Polyethylen, Laminate aus Polyethylen-Folie und Polyester-Folie, wobei als Polyester Polyethylenterephthalat besonders geeignet ist, Laminate aus Polyethylen-Folie und Folie aus Copolymeren von Ethylen und Vinylalkohol, Laminate aus Polyethylen-Folie und Folie aus Copolymeren aus Ethylen und Vinylacetat.

Die Herstellung der Verpackungseinheiten gemäß dieser Ausführungsform erfolgt derart, daß zunächst der Innenbehälter mit wenigstens einer Auslauftülle - wie vorstehend beschrieben - aus den vorstehend genannten Materialien hergestellt wird, dann - nach gegebenenfalls erfolgter Kreuzvernetzung - mit der aufzubewahrenden Flüssigkeit oder Lösung gefüllt und anschließend verschlossen wird. Anschließend wird der mit Flüssigkeit gefüllte und geschlossene Innenbehälter in an sich bekannter Weise mit dem Außenbehälter aus Polyamid 66 oder einem polymeren, Polyamid 66 aufweisenden Laminat, wobei im Falle der Verwendung eines Polyamid 66 aufweisenden Laminates für den Außenbehälter die dem Innenbehälter zugewandte Seite des Laminates aus Polyolefinfolie und die Außenseite des Laminates aus Polyamid 66-Folie besteht und die beiden Folien in üblicher Weise miteinander mittels eines Klebers verbunden sind, versehen und danach,wie vorstehend beschrieben,der Sterilisierung unterworfen.

Beispielsweise wird das Verfahren so durchgeführt, daß man den geschlossenen und mit der aufzubewahrenden Flüssigkeit oder Lösung gefüllten Innenbehälter in eine genau seinen Konturen entsprechende tiefgezogene Unterbahn für den Außenbehälter legt und mit der Oberbahn für den Außenbehälter bedeckt und anschließend an den Außenbehälter ein Vakuum anlegt, das etwa 900 bis 980 mbar betragen sollte. Gleichzeitig oder unmittelbar anschließend daran werden die Ränder von Oberbahn und Unterbahn des Außenbehälters miteinander verschweißt. Bei Anlegen des Vakuums legt sich die Folie oder das Laminat des Außenbehälters dicht an den Innenbehälter an. Die Unterbahn und die Oberbahn des Außenbehälters können aus gleichem oder verschiedenem Material bestehen und bestehen vorzugsweise aus gleichem Material. Ebenso können die Oberbahn und die Unterbahn des Außenbehälters gleiche oder verschiedene Stärke aufweisen und sind vorzugsweise gleich stark. Wesentlich ist, daß die Materialien von Unterbahn und Oberbahn - bei Laminaten die Innenschichten von Unterbahn und Oberbahn - miteinander verschweißbar sind. Wenn nur die Unterbahn tiefgezogen wird, sollte die Unterbahn vorzugsweise etwas stärker als die Oberbahn sein. Oberbahn und Unterbahn weisen geeigneterweise Stärken von 100 - 200 µm auf.

Die so hergestellte, mit der aufzubewahrenden Flüssigkeit oder Lösung gefüllte und verschlossene, Innen- und Außenbehälter aufweisende Verpackungseinheit wird anschliessend in einem Überdruckautoklaven bei einer Temperatur von etwa 120°C und einem Überdruck von etwa 2,2 bar sterilisiert.

Überraschenderweise wurde festgestellt, daß bei Verwendung von Außenbehältern, wie vorstehend beschrieben, insbesondere solchen aus einem Laminat aus einer Polyethylenfolie und einer Folie aus Polyamid 66, wobei die Polyethylenfolie auf der dem Innenbehälter zugewandten Seite des Laminates und auf der Außenseite des Laminates die Folie aus Polyamid 66 angeordnet ist und die beiden Folien in üblicher Weise mittels eines Klebers verbunden sind, nach der erfindungsgemäßen Verfahrensweise Verpackungseinheiten bzw. Behälter bereitgestellt werden, bei denen keine bzw. im wesentlichen keine Migration in die aufzubewahrende Flüssigkeit und keine Oxidation der aufzubewahrenden Flüssigkeit auftrat. Ebenso wurden keine Mikroorganismen gefunden.

Die nachfolgenden Beispiele dienen der weiteren Erläuterung der Erfindung.

### Beispiel 1

Aus einem Laminat aus Polyethylen und Polyamid 66, das dadurch hergestellt wurde, daß man eine handelsübliche Polyethylenfolie (Dichte 0,93 g/cm³) und eine handelsübliche Folie aus Polyamid 66 (Dichte 1,13g /cm³, Schmelzpunkt 255°C) mittels Polyurethan als Kaschierkleber miteinander verklebt hat, wurden Aufbewahrungsbeutel für eine Infusionslösung mit einem Fassungsvermögen von ca. 1 l hergestellt, wobei das Laminat unter Aussparung der Auslauföffnungen randverschweißt wurde und ein eine Verbindungsschicht aus einem Copolymerisat aus Ethylen und Vinylacetat mit einem Vinylacetatgehalt von 28 % aufweisendes Rohrstück aus Polycarbonat in die Auslauföffnung eingefügt und mittels Hitzesiegelung befestigt wurde. Bei diesen Beuteln stellt die Polyethylenfolie die Innenfolie und die Folie aus Polyamid 66 die Außenfolie dar. Der so hergestellte Beutel wurde mit der Infusionslösung gefüllt, verschlossen und in einen Überdruckautoklaven gebracht, wo er bei einer Temperatur von etwa 120° C und einem Überdruck von etwa 2,2 bar sterilisiert wurde. Das Laminat hatte eine Stärke von 180 µm, wobei die Polyethylenfolie 130 µm und die Polyamid 66-Folie 50 µm stark war.

Der so hergestellte Aufbewahrungsbeutel ist klarsichtig.

Bei Untersuchungen unter Verwendung dieses so hergestellten Beutels wurde festgestellt, daß in der in diesem Beutel enthaltenen Infusionslösung auch nach der Sterilisierung, wie vorstehend beschrieben durchgeführt, keine Fremdbestandteile in wägbaren Mengen (Wägegenauigkeit < 1 mg) zu finden waren.

Diese Untersuchungen wurden in der folgenden Weise durchgeführt:
Ein 1 l fassender Beutel wurde mit Wasser ad injection (1-fach Destillat, hochrein) gefüllt und bei 121°C (Haltezeit: 10 min) mit einem F₀ = 16 sterilisiert. Nach Abkühlen wurde der Beutelinhalt bei ca. 40°C am Rotationsverdampfer zur Trockne eingedampft und der eventuell erhaltene Rückstand ausgewogen.

Im Vergleich zum vorstehend gemäß Beispiel 1 hergestellten erfindungsgemäßen Beutel wurde bei einem in gleicher Weise hergestellten und aus gleichem Material - mit der Ausnahme, daß anstelle von Polyamid 66 Polyamid 6 verwendet wurde - bestehenden Beutel bei den gleichen Untersuchungen ein Rückstand an Fremdbestandteilen von 5 bis 15 ppm gefunden.

Diese Werte zeigen, daß bei dem erfindungsgemäßen Beutel gegenüber den Beuteln, die anstelle von Polyamid 66 Polyamid 6 aufweisen, keine Migration von Fremdbestandteilen in die Infusionslösung stattfand.

### Beispiel 2

Entsprechend dem in Beispiel 1 beschriebenen Verfahren wurde aus Polyethylenfolie (Stärke 130 µm) ein Innenbeutel mit einem Fassungsvermögen von ca. 1 l hergestellt, wobei die Folie unter Aussparung der Auslauföffnungen randverschweißt wurde und ein eine Verbindungsschicht aus einem Copolymerisat aus Ethylen und Vinylacetat mit einem Vinylacetatgehalt von 28 % aufweisendes Rohrstück aus Polycarbonat in die Auslauföffnung eingefügt und mittels Hitzesiegelung befestigt wurde. Der so hergestellte Beutel (Innenbeutel) wurde mit Infusionslösung gefüllt, verschlossen und - ohne vorheriges Sterilisieren - auf eine tiefgezogene Unterbahn eines Außenbeutels oder Umbeutels, deren Konturen genau denen des Innenbeutels entsprachen, gelegt und mit der Oberbahn bedeckt. Danach wurde ein Vakuum von 950 mbar an den Außen- oder Umbeutel angelegt und anschließend wurden die Unterbahn und die Oberbahn des Außen-oder Umbeutels entlang der Ränder miteinander verschweißt.

Die Oberbahn und die Unterbahn des Außen- oder Umbeutels bestanden jeweils aus einem Laminat aus Polyethylenfolie und einer Folie aus Polyamid 66, wie es für die Herstellung des Beutels gemäß Beispiel 1 verwendet wurde. Die dem Innenbeutel zugekehrte Folie des Laminates des Außenbeutels bestand aus Polyethylen und die Außenfolie des Laminates des Außenbeutels bestand aus Polyamid 66. Das Laminat für die Unterbahn hatte eine Dicke von 125 µm, wobei die Ethylenfolie eine Stärke von 75 µm und die Folie aus Polyamid 66 eine Stärke von 50 µm aufwies. Die Dicke des Laminates der Oberbahn betrug 125 µm, wobei die Polyethylenfolie eine Stärke von 75 µm und die Folie aus Polyamid 66 eine Stärke von 50 µm aufwies.

Die so hergestellte, mit Infusionslösung gefüllte und verschlossene, Innen- und Außenbeutel aufweisende Verpackungseinheit wurde anschließend in einen Überdruckautoklaven gebracht und dort bei einer Temperatur von etwa 120°C und einem Überdruck von etwa 2,2 bar sterilisiert.

Die erhaltene, den Außen- oder Umbeutel aus einem Laminat aus Polyethylenfolie und einer Folie aus Polyamid 66 aufweisende Verpackungseinheit war klarsichtig und gestattete auch nach Sterilisierung und langer Lagerung eine ausgezeichnete Sichtkontrolle für die im Beutel enthaltene Infusionslösung. In der von dem Innenbeutel und dem Außen - oder Umbeutel umgebenen Infusionslösung wurden - wie durch die vorstehend in Beispiel 1 beschrieben durchgeführte Untersuchung festgestellt wurde - auch nach der Sterilisation keine Fremdbestandteile in wägbaren Mengen (Wägegenauigkeit < 1mg) festgestellt. Die gelagerte Infusionslösung blieb klar. Es trat keine Migration von Fremdbestandteilen auf und wurden weder Oxidationsprodukte noch irgendwelche Mikroorganismen (Verpilzung oder Versporung) gefunden.

Untersuchungen - wie sie vorstehend beschrieben wurden - haben ergeben, daß demgegenüber bei einer gleichartig aufgebauten und aus gleichem Material hergestellten Verpakkungseinheit, wobei jedoch Polyamid 6 anstelle von Polyamid 66 für das Laminat für den Außen - oder Umbeutel verwendet wurde, in der Infusionslösung nach der Sterilisation ein Rückstand an Fremdbestandteilen von ca. 5 bis 15 ppm auftrat.

## Patentansprüche

1. Verpackungseinheit für medizinische Zwecke. insbesondere zur Aufnahme und/oder Lagerung von sterilisierbaren Parenteralien oder Dialyseflüssigkeiten, umfassend einen Behälter, der wenigstens eine Auslauftülle aufweist und aus einem polymeren Material besteht, dadurch gekennzeichnet, daß der Behälter nur aus Polyamid 66 besteht.

2. Verpackungseinheit für medizinische Zwecke, insbesondere zur Aufnahme und/oder Lagerung von sterilisierbaren Parenteralien oder Dialyseflüssigkeiten, umfassend einen Behälter, der wenigstens eine Auslauftülle aufweist und aus einem aus mit einem Kleber auf Polyurethanbasis verklebten Folien aus polymeren, Polyamid aufweisendem Material bestehenden Laminat aufgebaut ist, wobei die der aufzubewahrenden Flüssigkeit zugewandte Folie des Laminates aus Polyolefin besteht, dadurch gekennzeichnet, daß das Laminat aus einer der aufzubewahrenden Flüssigkeit zugewandten Polyolefinfolie und als Außenseite einer Folie aus Polyamid 66 besteht.

3. Verpackungseinheit nach Anspruch 2, dadurch gekennzeichnet, daß die Polyolefinfolie eine gegebenenfalls substituierte Polyethylen- oder Polypropylenfolie ist.

4. Verpackungseinheit nach Anspruch 2 oder 3, dadurch gekennzeichnet, daß das Laminat bis zu 0,2 mm stark ist, wobei die Polyolefinfolie eine Stärke von 50 bis 150 µm und die Folie aus Polyamid 66 eine Stärke von 20 bis 100 µm aufweist.

5. Verpackungseinheit nach einem der Ansprüche 1 bis 4, dadurch gekennzeichnet, daß der Behälter von einem Außenbehälter aus einem polymeren Material, insbesondere einem polymeren Laminat, umgeben ist.

6. Verpackungseinheit nach einem der Ansprüche 1 bis 5, dadurch gekennzeichnet, daß sie einen wenigstens eine Auslauftülle aufweisenden Innenbehälter umfaßt und der Behälter aus Polyamid 66 oder aus einem Polyamid 66 aufweisenden Laminat diesen Innenbehälter umschließt.

7. Verpackungseinheit nach Anspruch 6, dadurch gekennzeichnet, daß das Polyamid 66 aufweisende Laminat ein mittels eines Klebers verbundenes Laminat aus Polyamid 66 - Folie und Polyolefinfolie ist, wobei die Folie aus Polyamid 66 die Außenfolie und die Polyolefinfolie die dem Innenbeutel zugewandte Folie darstellt.

8. Verpackungseinheit nach Anspruch 6 oder 7, dadurch gekennzeichnet, daß der Innenbeutel aus Polyolefin, Polyestern und/oder Copolymeren derselben besteht.

## Claims

1. A package unit for medical purposes, in particular for receiving and/or storing sterilizable parenterals or dialysis solutions, including a container having at least one discharge spout and consisting of a polymeric material, characterized in that said container only consists of polyamide 66.

2. A package unit for medical purposes, in particular for receiving and/or storing sterilizable parenterals or dialysis solutions, including a container having at least one discharge spout and consisting of a laminate which consists of foils bonded together by means of an adhesive on the basis of polyurethane and made of polymeric material including polyamide, whereby the foil of the laminate facing the liquid to be stored consists of polyolefin, characterized in that the laminate consists of a polyolefin foil facing the liquid to be stored and as outer side of a foil made of polyamide 66.

3. A package unit according to claim 2, characterized in that the polyolefin sheet is a possibly substituted polyethylene or polypropylene sheet.

4. A package unit according to claim 2 or 3, characterized in that the laminate is up to 0.2 mm thick, whereby the polyolefin sheet has a thickness of from 50 to 100 µm and the sheet of polyamide 66 a thickness of from 20 to 100 µm.

5. A package unit according to any of claims 1 to 4, characterized in that the container is surrounded by an outer container which consists of a polymeric material, particularly a polymeric laminate.

6. A package unit according to any of claims 1 to 5, characterized in that it includes an inner container having at least one discharge spout and that the container of polyamide 66 or of a laminate containing polyamide 66 surrounds said inner container.

7. A package unit according to claim 6, characterized in that the laminate of polyamide 66 is a laminate of polyamide 66 sheet and polyolefin sheet which are connected by means of an adhesive, the sheet of polyamide 66 representing the outer sheet and the polyolefin sheet representing the sheet facing the inner bag.

8. A package unit according to claim 6 or 7, characterized in that the inner bag consists of polyolefin, polyesters and/or copolymers thereof.

## Revendications

1. Unité d'emballage à usage médical, en particulier pour la collecte et/ou la conservation de substances parentérales ou de liquides de dialyse stérilisables, comprenant un récipient qui comporte au moins une tubulure de sortie et consiste en une matière polymère, caractérisée en ce que le récipient consiste uniquement en polyamide 66.

2. Unité d'emballage à usage médical, en particulier pour la collecte et/ou la conservation de substances parentérales ou de liquides de dialyse stérilisables, comprenant un récipient qui comporte au moins une tubulure de sortie et qui est formé d'un stratifié consistant en pellicules de matière polymère présentant du polyamide collées avec un adhésif à base de polyuréthanne, la pellicule du stratifié tournée vers le liquide à conserver consistant en une polyoléfine, caractérisée en ce que le stratifié consiste en une pellicule de polyoléfine tournée vers le liquide à conserver et en une pellicule de polyamide 66 comme côté extérieur.

3. Unité d'emballage suivant la revendication 2, caractérisée en ce que la pellicule de polyoléfine est une pellicule de polyéthylène ou de polypropylène facultativement substitué.

4. Unité d'emballage suivant la revendication 2 ou 3, caractérisée en ce que le stratifié a une épaisseur jusqu'à 0,2 mm, tandis que la pellicule de polyoléfine a une épaisseur de 50 à 150 µm et la pellicule de polyamide 66 a une épaisseur de 20 à 100 µm.

5. Unité d'emballage suivant l'une quelconque des revendications 1 à 4, caractérisée en ce que le récipient est enveloppé d'un récipient extérieur d'une matière polymère, en particulier d'un stratifié polymère.

6. Unité d'emballage suivant l'une quelconque des revendications 1 à 5, caractérisée en ce qu'elle comprend un récipient intérieur comportant au moins une tubulure de sortie et le récipient en polyamide 66 ou en stratifié comportant un polyamide 66 enveloppe ce récipient intérieur.

7. Unité d'emballage suivant la revendication 6, caractérisée en ce que le stratifié comportant du polyamide 66 est un stratifié de pellicule de polyamide 66 et de pellicule de polyoléfine assemblé par un adhésif, la pellicule de polyamide 66 étant la pellicule extérieure et la pellicule de polyoléfine étant la pellicule tournée vers la poche intérieure.

8. Unité d'emballage suivant la revendication 6 ou 7, caractérisée en ce que la poche intérieure consiste en polyoléfine, en polyesters et/ou en copolymères de ceux-ci.
